# EUROPEAN PATENT SPECIFICATION

(11) **EP 0 286 239 B1**
(45) Date of publication and mention of the grant of the patent: **13.03.1996**
(21) Application number: 88302039.8
(22) Date of filing: 09.03.1988
(51) Int. Cl.: C12N 15/00, C12N 11/00, C12P 21/02

(54) **Production and purification of a protein fused to a binding protein**
Herstellung und Reinigung eines Proteins, das mit einem Bindungsprotein fusioniert ist
Production et purification d'une protéine fusionnée d'une protéine de liage

(30) Priority: 10.03.1987 US 24053
(43) Date of publication of application: 12.10.1988
(73) Proprietor: NEW ENGLAND BIOLABS, INC., Beverly Massachusetts 01915 (US); Temple University of the Commonwealth System of Higher Education, Philadelphia PA 19122 (US)
(72) Inventor: Guan, Chudi, Beverly Massachusetts 01915 (US); Inouye, Hiroshi, Deceased (US)
(74) Representative: Bass, John Henton

(56) References cited:
- EP-A- 0 157 235
- EP-A- 0 195 680
- EP-A- 0 244 147
- CHEMICAL ABSTRACTS, vol. 97, no. 17, 25th October 1982, page abstract no. 140527e, Columbus, Ohio, US; K. ITO.
- GENE, vol. 29, July 1984, pages 27-31, Amsterdam, NL; A. ULLMANN: "One-step purification of hybird proteins which have beta-galactosidase activity"
- Ito et al. (1982), Journal of Biological Chemistry Vol. 257 pp. 9895-9897.
- Bassford et al. (1979), Journal of Bacteriology, Vol. 139, pp. 19-31.

## Description

### BACKGROUND OF THE INVENTION

The present invention relates to a process of producing and/or purifying virtually any hybrid polypeptide or fused protein molecule employing recombinant DNA techniques. More specifically, a DNA fragment coding for a protein molecule, e.g. a polypeptide or portion thereof, is fused to a DNA fragment coding for a binding protein such as the gene coding for the maltose binding protein. The fused DNA is inserted into a cloning vector and an appropriate host transformed. Upon expression, a hybrid polypeptide or fused protein molecule is produced which can be purified by contacting the hybrid polypeptide with a ligand or substrate to which the binding protein has specific affinity, e.g. by affinity chromatography. The hybrid polypeptide so purified may in certain instances be useful in its hybrid form, or it may be cleaved to obtain the protein molecule itself by, for example, linking the DNA fragments coding for the protein molecule and binding protein with a DNA segment which codes for a peptide which is recognized and cut by a proteolytic enzyme. The present invention also relates to certain vectors useful in practicing the above process as well as to a bioreactor and methods employing the bound hybrid polypeptide, e.g. where the bound fused polypeptide is contacted and reacted with a susbstrate which interacts with the bound protein molecule to produce a desired result.

Recently developed techniques have made it possible to employ microorganisms, capable of rapid and abundant growth, for the synthesis of commercially useful proteins and peptides. These techniques make it possible to genetically endow a suitable microorganism with the ability to synthesize a protein or peptide normally made by another organism. In brief, DNA fragments coding for the protein are ligated into a cloning vector such as a plasmid. An appropriate host is transformed with the cloning vector and the transformed host is identified, isolated and cultivated to promote expression of the desired protein. Proteins so produced are then isolated from the culture medium for purification.

Many purification techniques have been employed to harvest the proteins produced by recombinant DNA techniques. Such techniques generally include segregation of the desired protein based on its distinguishing molecular properties, e.g. by dialysis, density-gradient centrifugation and liquid column chromatography. Such techniques are not universally applicable and often result in consumption of the purification materials which may have considerably more value than the protein being purified, particularly where substantial quantities of highly purified protein are desired.

Other procedures have been developed to purify proteins based on solubility characteristics of the protein. For example, isoelectric precipitation has been employed to purify proteins since the solubility of proteins varies as a function of pH. Similarly, solvent fractionation of proteins is a technique whereby the solubility of a protein varies as a function of the dielectric constant of the medium. Solvent fractionation, while giving good yields often causes denaturation of the protein molecule. Neither isoelectric precipitation nor solvent fractionation are useful in obtaining highly purified protein. Such techniques are typically employed in tandem with other procedures.

Proteins have also been separated based on their ionic properties by e.g. electrophoresis, ion-exchange chromatography, etc. Such electrophoretic techniques, however, have been used as analytical tools and are not practical as a means for purifying proteins on a large scale. Moreover, high purity and yield of the protein obtainable by such techniques is rarely achieved in a single step.

Affinity chromatography has also been employed in the purification of biopolymers such as proteins. Affinity chromatography involves a selective adsorbent which is placed in contact with a solution containing several kinds of substances including the desired species to be purified. For example, when used in protein purification protocols, affinity chromatography generally involves the use of a ligand which specifically binds to the protein to be purified. In general, the ligand is coupled or attached to a support or matrix and the coupled ligand contacted with a solution containing the impure protein. The non-binding species are removed by washing and the desired protein recovered by eluting with a specific desorbing agent. While affinity chromatography produces a relatively high level of purified protein, this technique requires significant amounts of the protein-specific ligand employed for purification. Moreover, the ligand will be different for each and every protein to be purified which necessarily entails a time-consuming and laborious regime. In addition, it has been found that specific ligands do not exist for all types of protein molecules, such as certain enzymes. As a result, affinity chromatography has not been successfully employed as a universal isolation purification technique for protein molecules.

One proposed attempt to universalize affinity chromatography to all proteins is described in European Patent Application 0,150,126 (Hopp). Disclosed is the preparation of a hybrid molecule produced by recombinant DNA techniques employing gene fusion. One gene codes for the desired protein to be purified while the other codes for an identification or marker peptide. The marker peptide contains a highly antigenic N-terminal portion to which antibodies are made and a linking portion to connect the marker peptide to the protein to be purified. The linking portion of the marker peptide is cleavable at a specific amino acid residue adjacent the protein molecule to be purified by use of a specific proteolytic agent. The fused or hybrid protein is isolated by constructing an affinity column with immobilized antibody specific to the antigenic portion of the marker peptide. The antibody binds to the fused protein which can thereafter be liberated from the column by a desorbing agent. The marker peptide may then be cleaved from the desired protein molecule with a proteolytic agent.

While purportedly overcoming some of the problems described above for protein purification protocols, Hopp requires substantial amounts of antibodies specific for the antigenic portion of the marker peptide. Moreover, the quantity of desorbing agent (in this case, a small peptide) required to compete off the target protein is substantial as well as a significant cost factor. Also, the desorbing agent must be purified away from the target protein. Thus, scale up for this system would not be practical. Furthermore, regeneration of the chromatographic column may be extremely difficult due to the destabilizing conditions employed to wash out the column after *use,* which may, in fact destroy the column. Others have suggested the use of low affinity antibody columns. However, low affinity columns often result in non-specific binding and would require significant cost for any large scale purification.

Thus, there is a continuing need for techniques which enable large scale purification of proteins produced through recombinant DNA processes without the above described problems. It would be particularly advantageous to provide an affinity purification process which utilizes an abundant and inexpensive ligand to which the fused protein would bind and an equally abundant and inexpensive desorbing agent.

### SUMMARY OF THE INVENTION

In accordance with the present invention there is provided a method for producing and highly purifying virtually any protein molecule generated by recombinant DNA techniques in a single affinity chromatography step. The method comprises:
(a) constructing a DNA expression vector which expresses a hybrid polypeptide in a transformed host cell, the hybrid polypeptide comprising the target protein molecule and a non-enzymatic biologically functional sugar binding protein, having a specific affinity for a substrate which binds to the non-enzymatic biologically functional sugar binding protein; and
(b) introducing the expression vector into an appropriate host cell and expressing the hybrid polypeptide;
(c) contacting the hybrid polypeptide produced by the transformed cell with the substrate to which the non-enzymatic biologically functional sugar binding protein binds; and
(d) recovering the target protein molecule.

The hybrid polypeptide or fused protein is produced by recombinant DNA techniques. The hybrid polypeptide can be isolated and purified directly, e.g. from the crude cellular extract or culture medium, simply by contacting the extract containing the hybrid polypeptide with a substrate to which the binding protein has specific affinity, e.g. using affinity chromatography. The bound hybrid polypeptide can easily be liberated from the column in a highly purified form with a desorbing agent which selectively desorbs the bound non-enzymatic sugar binding protein. While the target protein may be useful in its hybrid form, in certain preferred embodiments, it may be desirable to separate or cleave the non-enzymatic sugar binding protein away from the target protein. This may be accomplished in a variety of ways. For example, a DNA fragment coding for a predetermined peptide, e.g. a linking sequence, may be employed to link the DNA fragments coding for the binding and target proteins. The predetermined peptide is preferably one which is recognized and cleaved by a proteolytic agent such that it cuts the hybrid polypeptide at or near the target protein without interfering with the biological activity of the target protein. The linking sequence, in addition to providing a convenient proteolytic cleavage site, may also serve as a polylinker, i.e. by providing multiple DNA restriction sites to facilitate fusion of the DNA fragments coding for the target and binding proteins, and/or as a spacer which separates the target and binding protein which, for example, allows access by the proteolytic agent to cleave the fused polypeptide.

The preferred affinity column useful in practicing the present invention, in general, comprises a column containing immobilized ligand or substrate to which the binding protein has a specific affinity. As will be appreciated by the skilled artisan, the specific affinity of a binding protein for a given substrate will depend both on the particular binding protein employed as well as the substrate used in the column. In general, the substrate used in the column should bind substantially all of the particular binding protein without binding other proteins to which it is exposed. In certain instances, however, depending on the particular application (e.g. whether the column is used to purify the protein molecule or as a bioreactor for reacting the protein molecule with a substance with which it interacts to produce a desired result), a substrate may be used which only binds a portion of the binding protein present. In addition, the particular substrate employed should permit selective desorbtion of the bound binding protein with a suitable desorbing agent.

It will be appreciated that the column thus prepared can be used to isolate and purify virtually any protein which, by recombinant DNA techniques is linked to the binding, protein to form a hybrid polypeptide. The hybrid polypeptide can be released from the column with a suitable desorbing agent and/or cleaved with a proteolytic agent to separate the target protein from the binding protein. Alternatively, in accordance with another embodiment of the present invention, the bound hybrid polypeptide may be used as a bioreactor for reacting, for example, the biologically active portion of the protein molecule (which may be an enzyme, restriction endonuclease, etc.) with a substrate which interacts with the target protein. For example, if the target protein is an enzyme, the affinity column can serve as a means for immobilizing that enzyme, i.e. by the binding protein portion of the hybrid polypeptide being bound to the column. The substrate upon which the enzyme acts is thereafter passed through the column to achieve the desired result.

### BRIEF DESCRIPTION OF THE DRAWINGS

Figures 1 and 2 illustrate the construction of the maltose binding protein fusion cloning vector pCG150.

Figure 3 illustrates the DNA sequence of the polylinker region of the cloning vector pCG150.

Figure 4 illustrates the constuction of the mal E - Lac Z gene fusion plasmid pCG325.

Figure 5 illustrates elution profile of the protein resulting from affinity chromatography of a crude extract of SF1362/pCG325 containing the mal E - Lac Z fusion.

Figure 6 illustrates the activity profile of the protein resulting from affinity chromatography of a crude extract of SF1362/pCG325 containing the mal E - Lac Z fusion .

Figure 7 illustrates the SDS polyacrylamide gel electrophoresis of the product of the mal E - Lac Z fusion.

Figure 8 illustrates the native polyacrylamide gel electrophoresis of the product of the mal E - Lac Z fusion.

Figures 9 and 10 illustrate the construction of the mal E - Pst I restriction endonuclease gene fusion plasmid pCG410.

Figure 11 illustrates the SDS polyacrilamide gel electrophoresis of the product of the mal E - Pst I fusion.

### DETAILED DESCRIPTION OF THE INVENTION

The present invention provides a novel approach for producing and purifying a target protein molecule comprising:
(a) constructing a DNA expression vector which expresses a hybrid polypeptide in a transformed host cell, the hybrid polypeptide comprising the target protein molecule and a non-enzymatic biologically functional sugar binding protein, having a specific affinity for a substrate which binds to the non-enzymatic biologically functional sugar binding protein; and
(b) introducing the expression vector into an appropriate host cell and expressing the hybrid polypeptide;
(c) contacting the hybrid polypeptide produced by the transformed cell with the substrate to which the non-enzymatic biologically functional sugar binding protein binds; and
(d) recovering the target protein molecule.

The protein molecule is produced by constructing a DNA expression vector containing fused genes comprising a gene encoding the protein molecule and a gene coding for a non-enzymatic biologically functional sugar binding protein or portion thereof which has a specific affinity for a ligand or substrate and expressing the fusion in an appropriate host. The substrate is used as the matrix in an isolation/purification protocol, e.g. an affinity column, to recover the expressed product of the fused genes, i.e. the hybrid polypeptide. A DNA fragment which codes for a predetermined polypeptide can be used, e.g. flanking the gene coding for the binding protein,- in order to adjust the reading frame for the desired gene fusion and/or to introduce into the hybrid polypeptide a peptide sequence which is recognized and cleaved by a proteolytic agent which enables separation of the protein molecule from the binding protein where desired. As noted above, the bound hybrid polypeptide may also be used as a bioreactor for reacting the biologically active portion of the protein molecule with a substrate which interacts with the protein molecule.

The methods described herein by which DNA coding for a hybrid polypeptide is preferably cloned, expressed and purified include the following steps:

### I. Preparation of Fusion Vector.

A) The DNA encoding for the desired binding protein is purified.
B) The DNA is inserted into a cloning vector such as pBR322 and the mixture is used to transform an appropriate host such as E. coli.
C) The transformants are selected, such as with antibiotic selection or other phenotypic selection.
D) The plasmid DNA is prepared from the selected transformants.
E) The binding activity domain of the protein is determined and convenient restriction endonuclease sites are identified by mapping or created by standard genetic engineering methods.

### II. Insertion of DNA Coding for the Protein Molecule into the Fusion Vector.

A) The protein molecule gene is cloned by standard genetic engineering methods.
B) The protein molecule gene is characterized, e.g. by restriction mapping.
C) A DNA restriction fragment which encodes the protein molecule is prepared.
D) The protein molecule DNA fragment is inserted in the binding protein fusion vector so that an in-frame protein fusion is formed between the the DNA fragment coding for the binding protein and the DNA fragment coding for the protein molecule.
E) The vector containing this hybrid DNA molecule is introduced into an appropriate host.

### III. Expression and Purification of the Hybrid Polypeptide.

A) The host cell containing the fusion vector is cultured.
B) Expression of the fused gene is induced by conventional techniques.
C) A cell extract containing the expressed fused polypeptide is prepared.
D) The hybrid polypeptide is separated from other cell constitutants using an affinity column having as a matrix a substance to which the non-enzymatic biologically functional sugar binding protein part of the hybrid polypeptide has a specific affinity.
E) The bound purified hybrid polypeptide can be recovered and/or utilized by the following methods:
   (1) if the protein molecule's biological activity is maintained in its hybrid or fused configuration it may recovered from the column by eluting with a desorbing agent and used directly after elution in its hybrid form;
   (2) the protein molecule may be separated from the non-enzymatic biologically functional sugar binding protein either before or after elution from the column by proteolytic or chemical cleavage; and
   (3) the column may be used as a bioreactor with the fusion protein immobilized on the column, e.g. by contacting and reacting the bound fusion protein with a substrate which interacts with the biologically active portion of the protein molecule.

### Binding Protein

Non-enzymatic biologically functional sugar binding proteins which may be employed in accordance with the present invention include the sugar (e.g. mono-, di- or polysaccharide) binding proteins such as maltose or arabinose binding protein.

The preferred sugar binding protein for practicing the present invention is the maltose binding protein.

The product of the mal E Gene of E. coli, i.e. maltose binding protein (MBP) is a periplasmic osmotically shockable protein. MBP exhibits specific binding affinity with maltose and maltodextrins. Macromolecular alpha (1-4) linked glucans are also bound with high affinities. Ferenci, T. and Klotz, U. Escherichia Coli. FEBS Letters, Vol. 94, No. 2. pp. 213-217 (1978), the disclosure of which is hereby incorporated by reference. The dissociation constants are around 1um. Kellermann et al., Coli Eur. J. Biochem. 47. 139-149 (1974), the disclosure of which is hereby incorporated by reference. MBP is usually considered to exist as a monomer although it can exist as a dimer. Maltose induces the conversion of the dimer to the monomer. Gilbert, Biochemical and Biophysical Research Communications (1982) Vol. 105, No. 2, pp. 476-481, the disclosure of which is hereby incorporated by reference. MBP is a secreted protein which is synthesized in cytoplasm as a precursor with a 26 amino acid N-terminal signal peptide. Dupley, et al. J. Biol. Chem. Vol. 259 pp. 10606-10613 (1984), the disclosure of which is hereby incorporated by reference. During translocation across the cytoplasmic membrane the signal peptide is removed and the mature MBP is released into the periplasmic space. Mature MPB contains 370 amino acids corresponding to a molecular weight of 40,661 dalton( Dupley, et al., supra). MBP is made in large quantity in an induced culture (2-4x10⁴ monomers per cell). It has been determined that MBP and at least four other proteins make up the maltose transport system of E. coli. Shuman, J. Biol. Chem. 257: 5455-5461 (1982), the disclosure of which is hereby incorporated by reference. Besides being an essential component of the maltose transport system, MBP is also the specified chemoreceptor of the bacterium for maltose and maltodextrins. The Mal E gene has been cloned and sequenced. Dupley, et al., supra.

### Linking Sequence

A DNA fragment coding for a predetermined peptide may be employed to link the DNA fragments coding for the binding protein and protein molecule. The predetermined peptide is preferably one which is recognized and cleaved by a proteolytic agent such that it cuts the hybrid polypeptide at or near the protein molecule without interfering with the biological activity of the protein molecule. One such DNA fragment coding for a predetermined polypeptide is described in Nagai et al., Nature, Vol. 309, pp. 810-812 (1984), the disclosure of which is hereby incorporated by reference. This DNA fragment has the oligonucleotide sequence: ATCGAGGGTAGG and codes for the polypeptide Ile-Glu-Gly-Arg. This polypeptide is cleaved at the carboxy side of the arginine residue using blood coagulation factor Xa. As noted above the linking sequence, in addition to providing a convenient cut site, may also serve as a polylinker, i.e. by providing multiple restriction sites to facilitate fusion of the DNA fragments coding for the target and binding proteins, and/or as a spacing means which separates the target and binding protein which, for example, allows access by the proteolytic agent to cleave the hybrid polypeptide.

### Protein Molecule

The present invention may be beneficially employed to produce substantially any prokaryotic or eukaryotic, simple or conjugated protein that can be expressed by a vector in a transformed host cell. Such proteins include enzymes including endonucleases, methylases, oxidoreductases, transferases, hydrolases, lyases, isomerases or ligases.

The present invention also contemplates the production of storage proteins, such as ferritin or ovalbumin or transport proteins, such as hemoglobin, serum albumin or ceruloplasmin. Also included are the types of proteins that function in contractile and motile systems, for instance, actin and myosin.

The present invention also contemplates the production of antigens or antigenic determinants which can be used in the preparation of vaccines or diagnostic reagents.

The present invention also contemplates the production of proteins that serve a protective or defense function, such as the blood proteins thrombin and fibrinogen. Other protective proteins include the binding proteins, such as antibodies or immunoglobulins that bind to and thus neutralize antigens.

The protein produced by the present invention also may encompass various hormones such as Human Growth Hormone, somatostatin, prolactin, estrone, progesterone, melanocyte, thyrotropin, calcitonin, gonadotropin and insulin. Other such hormones include those that that have been identified as being involved in the immune system, such as interleukin 1, intereukin 2, colony stimulating factor, macrophage-activating factor and interferon.

The present invention is also applicable to the production of toxic proteins, such as ricin from castor bean or grossypin from cotton linseed.

Proteins that serve as structural elements may also be produced by the present invention; such proteins include the fibrous proteins collagen, elastin and alpha-keratin. Other structural proteins include glyco-proteins, virus-proteins and muco-proteins.

In addition to the above-noted naturally occuring proteins, the present invention may be employed to produce synthetic proteins defined generally as any sequences of amino acids not occurring in nature.

Genes coding for the various types of protein molecules identified above may be obtained from a variety of prokaryotic or eukaryotic sources, such as plant or animal cells or bacteria cells. The genes can be isolated from the chromosome material of these cells or from plasmids of prokaryotic cells by employing standard, well-known techniques. A variety of naturally occuring and synthetic plasmids having genes encoding many different protein molecules are now commercially available from a variety of sources. The desired DNA also can be produced from mRNA by using the enzyme reverse transciptase. This enzyme permits the synthesis of DNA from an RNA template.

### Preparation of DNA Fusion and Expression Vectors

Various procedures and materials for preparing recombinant vectors; transforming host cells with the vectors; replicating the vector and expressing polypeptides and proteins; are known by the skilled artisan and are discussed generally in Maniatis et al., Molecular Cloning: A Laboratory Manual, CSH 1982, the disclosure of which is hereby incorporated by reference.

In practicing the present invention, various cloning vectors may be utilized. Although the preferred vector is a plasmid, the skilled artisan will appreciate that the vector may be a phage. If cloning takes place in mammalian or plant cells, viruses can also be used as vectors. If a plasmid is employed, it may be obtained from a natural source or artificially synthesized. The particular plasmid chosen should be compatible with the particular cells serving as the host, whether a bacteria such as E. coli, yeast, or other unicellular microorganism. The plasmid should also have the proper origin of replication (replicon) for the particular host cell chosen. In addition, the capacity of the vector must be sufficient to accommodate the fusion coding for both the protein molecule of interest and the binding protein.

Another requirement for a plasmid cloning vector is the existence of restriction enzymes to cleave the plasmid for subsequent ligation with the foreign genes without causing inactivation of the replicon while providing suitable ligatable termini that are complementary to the termini of the foreign genes being inserted. To this end, it would be helpful for the plasmid to have single substrate sites for a large number of restriction endonucleases.

Moreover, the plasmid should have a phenotypic property that will enable the transformed host cells to be readily identified and separated from cells which do not undergo transformation. Such phenotypic selection genes can include genes providing resistance to a growth inhibiting substance, such as an antibiotic. Plasmids are now widely available that include genes resistant to various antibiotics, such as tetracycline, streptomycin, sulfa drugs, and ampicillin. When host cells are grown in a medium containing one of these antibiotics, only transformants having the appropriate resistant gene will survive.

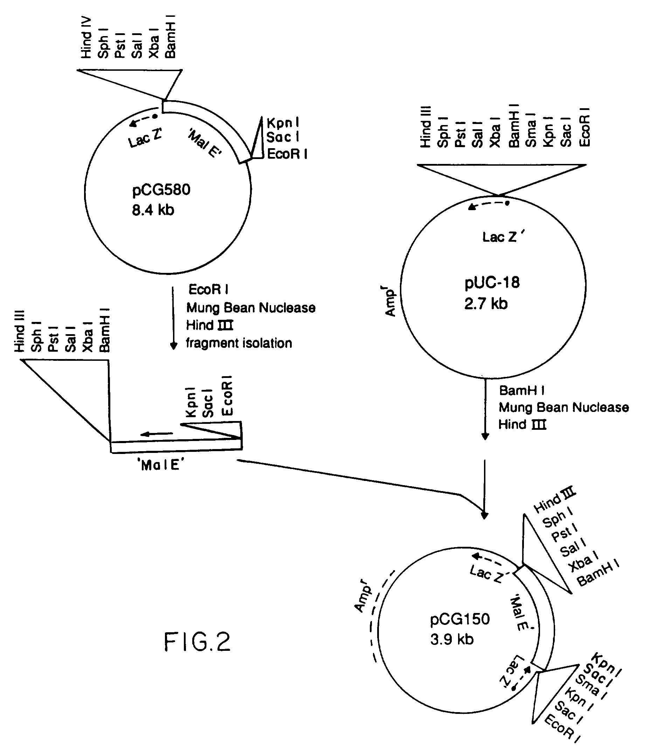

If E. coli is employed as the host cell, a preferred plasmid for performing the present invention is pCG150. A partial restriction endonuclease cleavage map of this plasmid is shown in Figure 2. An alternative plasmid for high level expression in E. coli is pCG806.

To prepare the chosen plasmid for ligation, preferably, it is digested with a restriction endonuclease to produce a linear segment(s) in which the two DNA strands are cleaved at closely adjacent sites to produce cohesive termini ("sticky ends") bearing 5ʹ-phosphate and 3ʹ-hydroxyl groups, thereby facilitating ligation with the foreign genes. For the plasmids identified above, restriction endonucleases will produce this result.

Certain restriction enzymes (Pvu II, Bal I) may result in the formation of blunt ends. The blunt ends of the plasmid can be joined to the foreign genes with T4 DNA ligase. The methods and materials for achieving efficient cleavage and ligation are well known in the art.

Prior to being joined with the selected cloning vector, it is desirable that the foreign genes coding for the binding protein and the protein molecule be first joined together. Ideally, the gene coding for the protein molecule molecule is treated with the same restriction endonuclease used to cleave the plasmid vector so that the appropriate termini of the gene will be compatible with the corresponding termini of the plasmid. This gene also may be treated with a second, different restriction endonuclease to prepare its opposite terminus for ligation with the binding protein gene.

The cointegrate genes are next ligated to the linearized plasmid fragment in a solution with DNA ligase. After incubation, the recircularized plasmid having the correct orientation of the cointegrate genes are identified by standard techniques, such as by gel electrophoresis.

### Transformation of Recombinant DNA Plasmid.

The recombinant DNA plasmids, as prepared above, are used for the transformation of host cells. Although the host cell may be any appropriate prokaryotic or eukaryotic cell, preferably it is well-defined bacteria, such as E. coli or yeast strain. Both such hosts are readily transformed and capable of rapid growth in fermentation cultures. In place of E. coli, other unicellular microorganisms can be employed, for instance fungae and algae. In addition, other forms of bacteria such as salmonella or pneumococcus may be substituted for E. coli. Whatever host is chosen, it should be one that has the necessary biochemical pathways for phenotypic expression and other functions for proper expression of the hybrid polypeptide. The techniques for transforming recombinant plasmids in E. coli strains are widely known. A typical protocol is set forth in Maniatus et al. supra.

In transformation protocols, only a small portion of the host cells are actually transformed, due to limited plasmid uptake by the cells. Thus, before transformants are isolated, the host cells used in the transformation protocol typically are multiplied in an appropriate medium. The cells that actually have been transformed can be identified by placing the original culture on agar plates containing a suitable growth medium containing the phenotypic identifier, such as an antibiotic. Only those cells that have the proper resistance gene will survive. Cells from the colonies that survive can be lysed and then the plasmid isolated from the lysate. The plasmid thus isolated can be characterized, e.g. by digestion with restriction endonucleases and subsequent gel electrophoresis or by other standard methods.

Once transformed cells are identified, they can be multiplied by established techniques, such as by fermentation. In addition, the recovered cloned recombinant plasmids can be used to transform other strains of bacteria or other types of host cells for large scale replication and expression of the fused protein.

### Purification of the Fused Protein

The hybrid polypeptide expressed by the transformed host cell are preferably separated from all other cellular constitutents and growth media by an affinity chromatography process. The column matrix is simply any substrate for which the binding protein has specific affinity. For example, when the binding protein is MBP the column matrix may be crosslinked amylose. Crosslinked amylose prepared by an epichlorohydrin protocol satisfies the substrate specificity of MBP and provides a rapid one step chromatographic purification of MBP from osmotic-shock fluids, Ferenci, T. et al., supra, whole cell extracts or culture media.

An extract from the transformed host cell is contacted with the column to isolate the hybrid polypeptide. The hybrid polypepetide may thereafter be eluted from the column, for example, by adding a dilute solution of a desorbing agent which displaces the hybrid polypeptide.

### Separation of the Protein Molecule from the Hybrid Polypeptide

The hybrid polypeptide purified from the above affinity column may be cleaved by sequence specific proteases such as a factor Xa or by discrete chemical cleavage such as cyanogen bromide.

The following examples are given to additionally illustrate embodiments of the present invention as it is preferred to practice. It should be understood that these examples are illustrative, and that the invention is not to be considered as restricted thereto except as indicated in the appended claims.

### EXAMPLE I

Example I describes cloning, expression and purification of B-galactosidase as a product of the mal E - Lac Z gene fusion.

### Preparation of the Binding Protein Fusion Vector

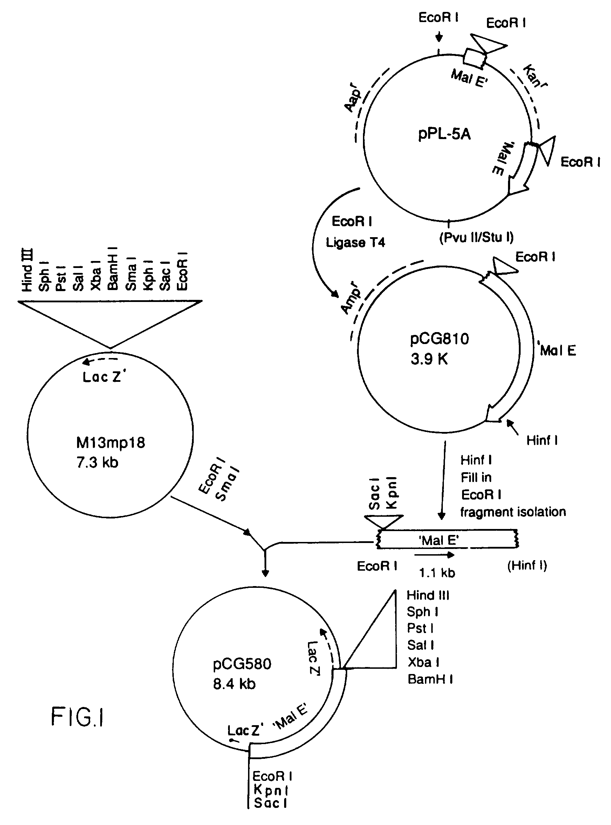

Plasmid pPL-5A is the source for the Mal E encoding DNA fragment which is prepared by first creating a deletion derivative of pPL-5A which moves the Mal E promoter and signal sequence. This plasmid is pCG810. The gene encoding Mal E is then resected from pCG810 and inserted into M13mp18 to produce recombinant phage pCG580, which has added multiple cloning sites to facilitate insertion of protein molecule encoding DNA. The Male E gene now carrying the additional cloning site is resected from pCG580 and inserted into pUC18 in order to create additional cloning sites as well as pick up a selective antibiotic resistance gene. The resulting plasmid is the protein fusion vector pCG150 which contains the Mal E gene and additional cloning sites and which is used in the construction of the vector which also contains the DNA coding for the desired protein molecule, infra. A sample of pCG150 has been deposited with the American Type Culture Collection under ATCC accession No. 67345. The construction of plasmid pCG150 is illustrated in Figs. 1 and 2.

According to the published Mal E gene sequence of E. coli there are five Taq I recognition sites in the gene. One is located at base number 83-86 (Dupley, et al. supra) corresponding to the second and third codon of mature maltose binding protein (MBP) coding sequence. A kanamycin resistance determinant fragment flanked by polylinkers was inserted into this Taq I site. The resulting plasmid was pPL-5A.

5-10 ug of pPL-5A plasmid DNA and 10 units of EcoRI restriction enzyme in 100ul of EcoRI digestion buffer was incubated for 2 hours at 37°C. 20ul of DNA gel loading buffer (0.25% bromophenol blue, 40mM EDTA, pH 8.0, 30% glycerol) were added and mixed. The digested sample was applied to 1% low gelling temperature agarose gel (Seaplaque). Gel electrophoresis was performed at low current (20mA) for 4 hours. TEA gel electrophoresis buffer (40mM Tris-acetate, pH 8.0. 2mM EDTA) was used. The gel was stained with TEA buffer containing ethidium bromide 0.5 ug/ml for 30 minutes at room temperature. Three DNA bands were visualized on the gel by U.V. irradiation. The largest fragment was cut out of the gel and placed in a 1.5 ml microfuge tube. The tube was incubated for 5 minutes in a 65°C water bath. The melted gel (about 100ul) was extracted with an equal volume of phenol and phenol/chloroform and chloroform as described by Maniatis et al, supra, at page 170, the disclosure of which is hereby incorporated by referernce. The aqueous phase was saved and 1/10 volume of 3N sodium-acetate pH 5.5 was added and mixed. 2.5 volumes of ethanol was added. The ethanol precipitate mixture was placed in -70°C freezer for 20 minutes (or in -20°C freezer overnight), then centrifuged for 15 minute in a microfuge at 4°C. The supernatant was discarded and the pellet was rinsed with 0.5 ml of 70% ethanol twice. The tube was left open at room temperature to eliminate any remaining ethanol. The DNA pellet was dissolved in 19 ul of water followed by adding 4 ul of 6x ligation buffer (300mM Tris-HCl pH 7.4, 60mM Mg Cl₂, 60mM dithiothreitol, 6 mM ATP, 600ug BSA) and 1ul of T4 DNA ligase (10 units) and incubated at 16°C overnight. The ligation solution was used to transform competent cells of E. coli strain SF 1362. The competent cells were made and the transformation was performed as described by T.J. Silhavy et al., in Experiments with Gene Fusions, CSH pp. 169-170 (1984), the disclosure of which is hereby incorporated by reference. After heat shock the transformation mixture was incubated with 5 ml LB medium for 45 minutes at 37°C. The cells were collected by centrifugation for 5 minutes at 3000 r.p.m. and resuspended in 0.5 ml of LB medium. 0.05-0.2 ml of the cells were spread on LB plates containing ampicillin 100 ug/ml. After overnight incubation at 37°C a total of about 1000 transformants were obtained. 16 transformants were purified on the same plates. Plasmid DNA minipreparations from the purified transformants were performed as described by Silhavy et al., supra. Restriction enzyme analysis on the plasmid DNAs was also performed. One plasmid was chosen, pCG810, in which the kanamycin resistance determenent sequence and the malE promotor and signal sequence regions had been deleted and the single EcoR,I BglII, BssHII and NcoI cutting sites remained.

10-20 ug of plasmid pCG810 DNA prepared and purified by the BND cellulose procedure described by Gamper et al., DNA, Vol. 4, No.2 (1985), the disclosure of which is hereby incorporated by reference, and 20 units of Hinf I restriction enzyme in 100ul of Hinf I digestion buffer (recommended by N.E.B.) were incubated for 2 hours at 37°C then extracted with phenol and chloroform and precipitated with ethanol as described above. The DNA was dissolved in 50 ul of the filling in reaction buffer (50mM Tris. pH 7.4. 10mM MgCl₂, 1mM dithiothreitol, 0.1mM dATP, 0.1mM dCTP, 0.1mM dGTP and 0.1mM dTTP containing 5 units of DNA polymerase I large fragment and incubated for 20 minutes at room temperature. 50 ul of TE buffer (10mM Tris. pH 8.0, 1mM EDTA) were added and extracted with phenol and chloroform and the aqueous phase precipitated with ethanol. The DNA was cleaved with EcoRI restriction enzyme in 100 ul of EcoRI digestion buffer followed by ethanol precipitation. The DNA was redissolved in 50 ul of TE followed by 10 ul of DNA gel loading buffer and applied to 1% of low gelling temperature agarose gel. The gel electrophoresis and DNA extraction from gel were as described above. The 1.1 kb EcoRI-Hinf I fragment which contained almost the entire MBP coding sequence was purified and dissolved in 10 ul of DNA buffer (10mM Tris pH 8.0, 0.1mM EDTA), stored at -20°C.

5 ug of M13mp18 double stranded DNA (Yanisch-Perron et al., Gene: 33, pp.103-119 at 104, (1985)), the disclosure of which is hereby incorporated by reference, and 10 units of SmaI restriction enzyme in 50 ul of SmaI digestion buffer were incubated for 30 minutes at 37°C followed by phenol extraction and ethanol precipitation as described above. The digested DNA was then dissolved in 50 ul of EcoRI digestion buffer containing 10 units EcoRI restriction enzyme and incubated for 1 hour, then extracted with phenol and chloroform, precipitated with ethanol as described above. The DNA pellet was dissolved in 10 ul of DNA buffer.

Two DNA preparations, the 1.1 kb EcoRI-HinfI fragment and the EcoRI and SmaI digested M13mp18 vector, were pooled and ligation was performed as described above. The ligation solution was used to transform JM101 or 71-18 competent cells (Yanisch-Peron et al., supra). The transformation was done as described above. After the heat shock the cells were mixed with JM101 or 71-18 exponentially growing cells and melted soft agar keeped at 47°C and plated on LB plates containing XG and IPTG described by J. Messing in NIH Publication No. 79-99, Vol. 2, (1979) at 43-48, the disclosure of which is hereby incorporated by reference. About 500 to 1000 plaques appeared on the plate; 60% were white, 40% blue. About 100 white plaques were picked up with sterile pasteur pipets and added to 5 ml culture tubes containing 2 ml early log phase culture of JM101 or 71-18. The tubes were incubated for 5-6 hours at 37°C with shaking. The phage containing supernatants were seperated from the cells by transfering 1 ml each of culture into a microfuge tube and centrifugation for 10 minutes with microfuge at room temperature. 20 ul of supernatant were withdrawn and mixed with 1 ul of 2% S.D.S. and 4 ul of DNA gel loading buffer. Samples were electrophoresed through 0.8% agarose gel in 4xTAE buffer overnight. The recombinant phages were identified by slower migration through the gel as compared with single stranded DNA of phage M13mp18. Double stranded DNAs were made from the recombinant phages and restriction enzyme analyses were carried out. One recombinant phage pCG580 was chosen which had the Mal E gene sequence insertion in the same direction as Lac Z gene on M13mp18, in which the EcoRI cutting site was regenerated. The BamHI-XbaI-SalI-PstI-SphI-HindIII polylinker remained. BglII, BssHII and NcoI cutting sites were introduced in by the insertion of the malE sequence.

5 ug of pCG580 double stranded DNA purified with BND cellulose was cleaved with EcoRI restriction enzyme followed by blunting the cohesive ends with DNA polymerase I large fragment as described above. The DNA was religated and used to transform JM101 or 71-18. Only less than 5% of transformants were blue. It seemed that the filling in EcoRI cutting site created an in-frame TAA codon which could not be suppressed by Sup E carried by JM101. The small portion of blue transformants could be explained by a base deletion from the cohesive ends during the DNA manipulation and indicated the inserted Mal E sequence was in the same reading frame with down stream Lac Z sequence since no detectable DNA deletion was found for the plasmids made from the blue transformants by restriction enzyme analyses.

10-20 ug of double stranded pCG580 DNA purified with BND cellulose was cleaved with EcoRI. After phenol extraction and ethanol precipitation the DNA pellet was dissolved in 100 ul of mung bean exonuclease buffer containing about 5 units mung bean exonuclease and incubated for 20 minutes at 37°C followed by phenol extraction and ethanol precipitation. The blunted DNA was then cleaved with Hind III restriction enzyme in 50 ul of Hind III digestion buffer. This sample was electrophoresed through 1% of low gelling temperature agarose gel. The 1.1 kb DNA fragment containing MBP coding sequence tailed with polylinker was purified from the gel as described above. The purified DNA fragment was stored in 10 ul of DNA buffer at -20°c.

10 ug of pUC-18 plasmid DNA and 20 units of BamH1 restriction enzyme in 100 ul of BamH1 digestion buffer were incubated for 1-2 hours at 37°C. After phenol extraction and ethanol precipitation the digested DNA was treated with mung bean exonuclease to blunt the cohesive ends as described above. After phenol extraction and ethanol precipitation the DNA was dissolved in 10 ul of DNA buffer.

Two DNA preparations, the 1.1 kb fragment from pCG580 and the BamHI cleaved pUC-18, were pooled and 4 ul of 6x ligation buffer and 1 ul of T₄ ligase (5-10 units) were added and mixed. The ligase solution was incubated overnight at 16°C followed by incubation for 4 hours at room temperature and used to transform JM103 or 71-18. Transformants were selected on LB plates containing ampicillin 100 ug/ml. Recombinant plasmids were identified by the size of DNA with the toothpick assay as described by Shinmick et al., Nucl. Acids Res. Vol. 2, p. 1911, the disclosure of which is hereby incorporated by reference. About 12 recombinant plasmids were scored and three produced blue color on LB ampicillin plates in the presence XG and IPTG. One was chosen as plasmid pCG150. 5 ug of pCG150 plasmid DNA purified with BND cellulose was cleaved with EcoRI restriction enzyme followed by blunting the cohesive ends with large fragment DNA polymerase I, then ligated with T₄ Ligase. When this DNA was used to transform JM101 or 71-18, more that 95% of transformants were white in presence of XG and IPTG. This indicated no translation restarted in the downstream Mal E gene region.

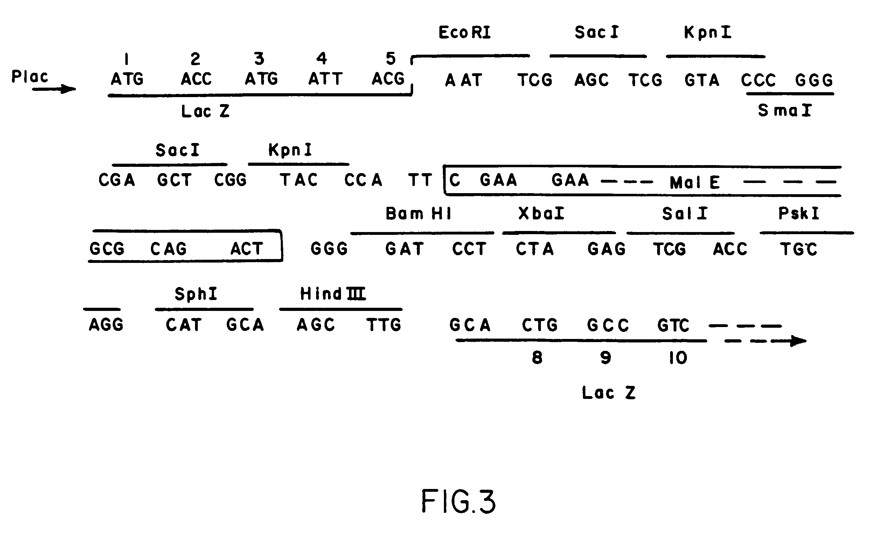

The Mal E gene joint regions on plasmid pCG150 were sequenced and the results presented in Fig 3.

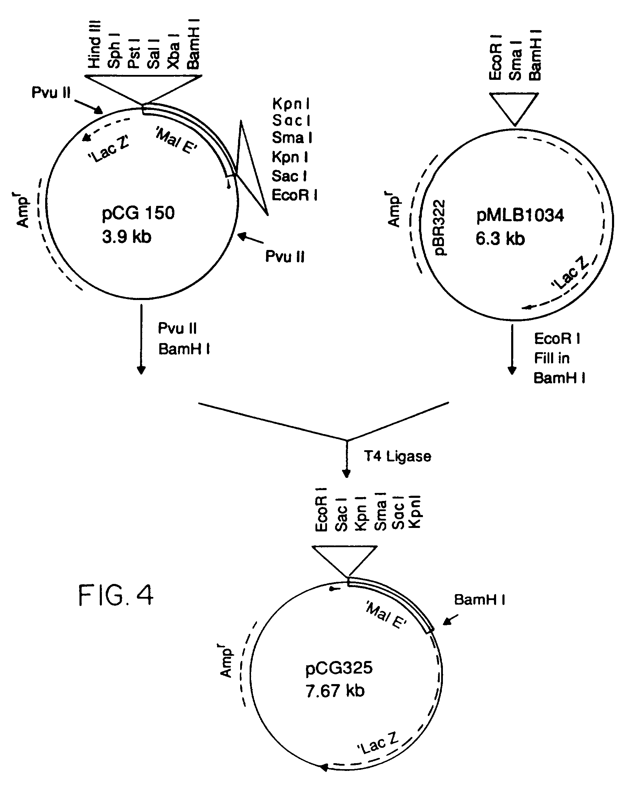

The Mal E - B-galactosidase fusion protein plasmid pCG325 illustrated in Fig. 4 was constructed as follows. Plasmid pMLB1034 was constructed by Silhavy et al, supra. This plasmid contains the Lac Z gene coding for B-galactosidase without the promotor or first 8 codons of the protein and a polylinker containing EcoRI, SmaI and BamHI restriction sites. 5 ug of pMLB1034 was cleaved with EcoRI restriction enzyme followed by blunting the cohesive ends with DNA polymerase large fragment, then cleaved with BamHl. After phenol extraction and ethanol precipitation the DNA was dissolved in 10 ul of DNA buffer and stored at -20°C.

5 ug of pCG150 DNA was cleaved with BamHl and PVUII restriction enzymes, extracted with phenol chloroform, precipitated with ethanol. The DNA was dissolved in 10 ul of DNA buffer. Two pCG150 and PMLB1034 DNA preparations were pooled and ligated as described above. The ligation solution was used to transform competent cells made from an E. coli straim MC4100 Silhavy, T.J., et al, supra and spread on LB plates containing ampicillin 100 ug/ml, XG 20 ug/ml. After overnight incubation several hundred transformants appeared on plates, 20-30% of them were blue. About 24 blue transformants were purified and used to isolate plasmid DNAs usingh the rapid isolation method described by Silhavy, supra. Restriction enzyme analyses were performed on these plasmid DNAs.

One recombinant, plasmid pCG325, was chosen and characterized. This plasmid contained the 1.3kb Mal E gene sequence from pCG150 which had been inserted in the EcoRI-BamHl site of pMLB1034.

### Affinity Chromatography

A double deletion (ΔLacΔmalB) strain E. coli (SF1362) habouring pCG325 was grown to late log phase in rich medium containing ampicillin 100 ug/ml. Cells were harvested by centrifugation with a Beckman centrifuge for 15 minutes at 5000 r.p.m. at 4°C. 5 gms of harvested cells were washed with 100 ml of 10mM TRIS. pH 7.2 at 4°C, then resuspended in 50 ml of the same buffer. Cells were broken by sonication at 4°C. Cell debris was separated by centrifugation with a Beckman centrifuge for 30 minutes at 16000 r.p.m. The supernatant was dialysed against 1 L of the same buffer for 3-4 hours at 4°C. A sample was applied onto a 3 x 5 cm cross-linked amylose column prepared as described by Ferenci et al., supra at pp. 459-463.

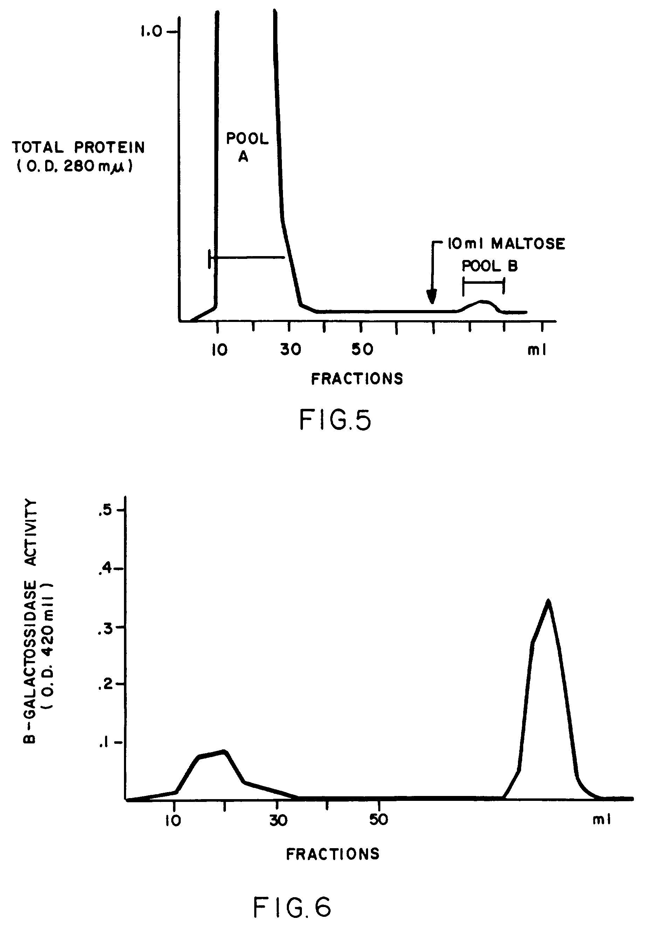

After the major 280 mu absorbant peak passed through at about 20-30 ml the column was extensively washed with 10-20 column volume of 10mM Tris pH 7.2. The column was eluted with 10mM Tris, pH 7.2, containing 10mM maltose. Both O.D 280mu and B-galactosidase activity (Miller, Experiments in Molecular Genetics, CSH (1972), pp. 325-355, the disclosure of which is hereby incorporated by reference) were measured for each fraction. The eluting profiles are illustrated in Figure 5. Figure 6 shows that more than 95% of OD280 absorbing material in the crude extracts passed through the column. Only less then 1% was retained by the column and could be eluted with 10mM maltose buffer. In contrast more than 70% of B-galactosidase activity was retained by the column and eluted with 10mM maltose (Figs. 5 and 6). When the pass through fractions were pooled and reapplied onto another cross-linked amylose column, the B-galactosidase activity present in these fractions was not retained. This suggests that a small portion of the hybrid polypeptide was degraded to such a degree that the degraded products lost binding activity with cross-linked amylose, but still maintained some B-galactosidase, enzymatic activity. When the maltose eluted fractions were dialysed and pooled and reapplied onto another cross-linked amylose column, the B-galactosidase activity present in these fractions was, retained and could be eluted with 10mM maltose buffer.

### Polyacrylamide Gel Electrophoresis

Affinity chromatography peaks were pooled separately. The maltose eluted peak was concentrated 25-50 fold. 20-40 ul of concentrated sample were mixed with double strength loading buffer (0.5 M Tris-HCl, pH 6.8, 30% glycerol, 4% SDS, 6% beta-mercaptoethanol, 0.4% bromophenol blue) and boiled for two minutes. Samples were applied onto 7 or 10% polyacrylamide gel (29:1). The electrophoresis buffer system was used as described by Laemmli, Nature, Vol. 227, pp. 680-685 (1970), the disclosure of which is hereby incorporated by reference. The gel electrophoresis was performed at 7-10 V/cm or 20 mA for 5 to 7 hours followed by staining with Coomasie Brillant blue R 250 (0.1% coomasie blue, 50% methanol, 10% acetic acid. The gels were destained with destaining solution of 10% acetic acid and 10% methanol).

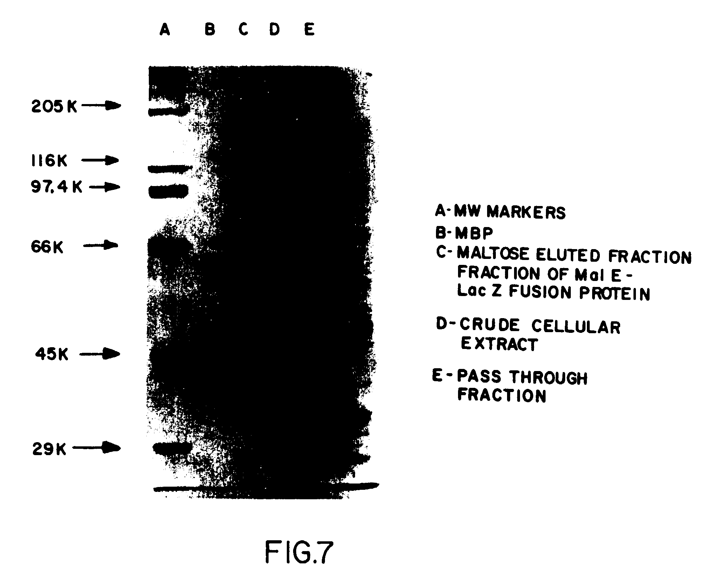

The results of SDS gel electrophoresis are shown in Figure 7. It appeared that almost all of the protein in the crude extract passed through the column. Only the hybrid polypeptide and small particles of its degraded products were retained by the column and eluted with maltose buffer. The main band on the gel represents the hybrid polypeptide whose molecular weight is estimated at 156k, corresponding to that deduced from the gene fusion sequence.

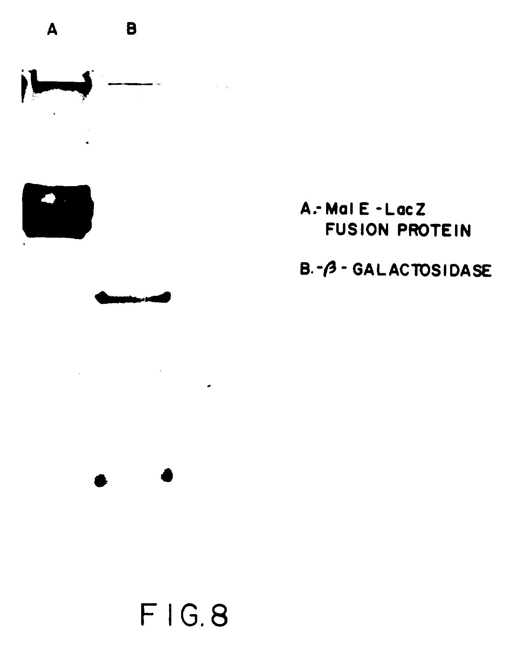

Native protein gel analysis was also carried out. For native gels the SDS was omitted from the electrophoresis buffer system and the electrophoresis gel was rinsed with water then covered with Z buffer 0.1M NaP04 pH 7.0, KCl 0.01M, Mg2SO4, 0.001M, B-Mercaptoethanol 0.05M) containg XG 20 ug/ml and incubated for 4 hours at 37°C without shaking. When the blue band appeared on gel, the buffer was discarded. This shows that the hybrid polypeptide, which migrated slower than the native B-galactosidese, represents the B-galactosidase enzymatic activity in the maltose buffer eluted fraction (Figure 8).

### Immunodiffusion Experiment

Double immunodiffusion (Ouchterlony) experiment was performed on 1% agarose gel in the buffer 10mM Tris, pH 7.2 150mM NaCl. 5-10 ug of sample protein were used (Anti MBP sera obtained from Jon Beckwith of Harvard Medical School. Anti B-galactosidase sera was obtained from Promega Biotech, WI. The purified hybrid polypeptide formed precipitation lines with both anti MBP sera and anti B-galactosidase sera. Pure B-galactosidase formed a precipitation line only with anti B-galactosidase sera and the maltose binding proteins only with anti MBP sera.

### EXAMPLE II

Example II describes the cloning, expression and purification of PstI restriction endonuclease as a product of the Mal E-Pst I restriction gene fusion.

### Recombinant DNA

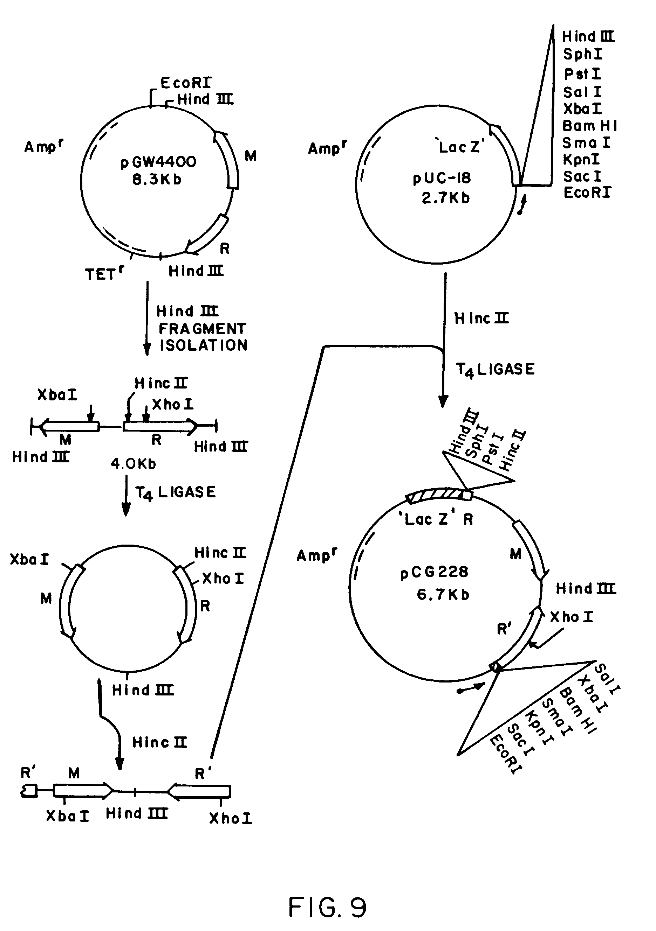

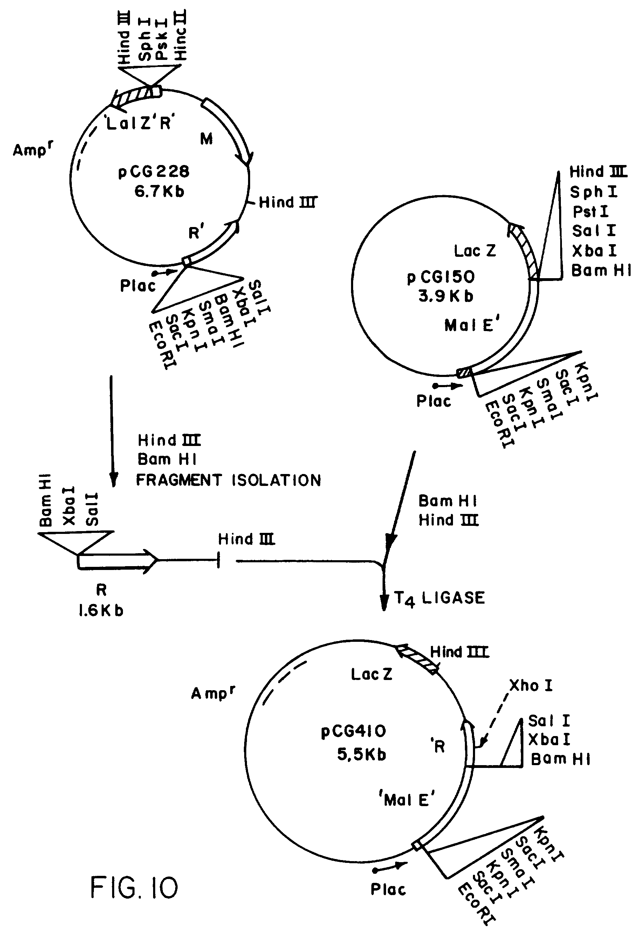

The outline of construction of plasmid pCG410 is illustrated in Fig. 9 and 10.

According to the published DNA sequence of Pst I restriction and modification system described in Walder et al., J. Biol. Chem Vol. 259 No. 12, pp. 8015-8026 (1984), the disclosure of which is hereby incorporated by reference, the restriction gene and the methylase gene are transcribed divergently from the promoter region between the two genes. There is a Hinc II restriction enzyme cleavage site at the eighth codon of the Pst I restriction gene. A Hind III DNA fragment (4.0kb) containing Pst I restriction and modification genes has been cloned in the Hind III site of plasmid pBR322. This plasmid is pGW4400.

30 ug of plasmid pGW440 DNA were cleaved with 30 units of Hind III restriction enzyme and 30 units of Pvu II restriction enzyme in 200 ul of Hind III digestion buffer followed by phenol/chloroform extraction and ethanol precipitation. The DNA was dissolved in 50 ul of TE buffer followed by mixing with 10 ul of loading buffer. A sample was elecrophoresed through 1% of low gelling temperature agarose. After electrophoresis the gel was stained with ethidium bromide and the DNA bands were visualized with UV irradiation as described in Example I. Three bands appeared on gel. The topmost one (4.0kb) was cut out and the DNA was extracted from gel as described in Example I. The purified DNA fragment was ligated with 50 units of T4 DNA Ligase in 0.5 ml of ligation buffer followed by phenol/chloroform extraction and ethanol precipitation. The DNA was cleaved with 30 units of Hinc II restriction enzyme in 100 ul of Hinc digestion buffer followed by phenol/chloroform extraction and ethanol precipitation. The DNA was dissolved in 20 ul of DNA buffer.

5 ug of plasmid pUC18 DNA was cleaved with 10 units of Hinc II restriction enzyme followed by phenol/chloroform extraction and ethanol precipitation. The DNA was dissolved in 10 ul of DNA buffer.

Two DNA preparations, the 4.0 kb fragment from pGW4400 and the Hinc II cleaved pUC-18, were pooled, followed by adding 5 ul of 6x ligation buffer and 2 ul (or 10 units) of T4 ligase and incubated overnight at room temperature. The ligation solution was used to transform competent cells of JM 101 as described in Example I. The transformation mixture was plated on LB plates containing ampicillin 100 ug/ml, XG 20 ug/ml and IPTG 10-4M. After overnight incubation about 100 transformants were obtained. 20% of them were white. 32 white transformants were purified and DNA minipreparations were made from the white transformants as described in Example I. The recombinant plasmids were identified by restriction enzyme analysis. One recombinant plasmid was chosen as pCG228 whose construction is presented in Figure 9.

10-20 ug of plasmid pCG228 DNA purified with BND cellulose were cleaved with 20 units of BamH I restriction enzyme and 20 units of Hind III restriction enzyme in 100 ul of the BamH I-Hind III double digestion buffer (10mM Nacl, 3mM dithiothrietol 10mM MgCl2). The 1.6 kb BamHi-HindIII DNA fragment contained the Pst I restriction gene whose promoter and first 7 codons had been replaced by a BamHi-XbaI-SalI polylinker. This fragment was purified from low gelling temperature agarose gel as described in Example I. The purified DNA fragment was dissolved in 10 ul of DNA buffer.

10 ug of plasmid pCG150 were cleaved with BamH I and Hind III restriction enzymes followed by phenol/chloroform extraction and ethanol precipitation as described above. The DNA was dissolved in 10 ul of DNA buffer.

The two DNA preparations, the 1.6 kb BamH I-Hind III fragment and pCG150 cleaved vector, were pooled and ligated with 10 units of T4 DNA Ligase in 30 ul of ligation buffer by incubation of the ligation solution overnight at 16°C. The ligation solution was used to transform competent cells of MC4100 habouring plasmid pACYC184 (Lac I),. pACYC184 (Lac I) (Chang, et al., J. Bact. Vol.134 No.3 pp.1141-1156 (1978), the disclosure of which is hereby incorporated by reference) is a multicopy plasmid and is compatible with plasmid pBR322 in E. coli K12. A DNA fragment containing the Lac I gene was inserted into the EcoR I cutting site of pACYC184. This is plasmid pACYC184 (Lac I). In order to prepare competent cells of MC4100 harbouring pACYC184 (Lac I), MC4100 was first transformed with plasmid pACYC184 (Lac I). The transformants (tetracycline resistant) were then used to prepare competent cells as described in Example I. These are competent cells of MC4100 harbouring pACYC184 (Lac I). The transformation mixture was placed onto LB plates containing ampicillin, 100 ug/ml, tetracycline 20 ug/ml. About 50-100 transformants appeared on each plate after overnight incubation. The plates were replicated onto LB plates containing ampicillin 100 ug/ml, tetracycline 20 ug/ml and IPTG 4x10-4 M. The replicated plates were incubated overnight at 37°C. The transformants which grew on LB-ampicillin-tetracycline plates but failed to grow on LB-ampicillin-tetracycline-IPTG plates were saved and purified on LB-ampicillin-tetracycline plates. DNA mini-preparations were made from the IPTG sensitive transformants and used to transform JM103 or 71-18. The transformants which were resistent to ampicillin but sensitive to tetracycline and 10⁻⁵M IPTG were saved. DNA mini preparations were made from these IPTG sensitive transformants and analyzed with restriction enzyme digestions. One recombinant plasmid was chosen as pCG410 whose construction is presented in Figure 10.

### Affinity Chromatography of Pst I - Mal E Fusion

E. coli strain MC4100 harbouring both plasmids pCG410 and pACYC184 (Lac I) was cultivated to late log phas in rich medim containing ampicillin 100 ug/ml and tetracycline 20 ug/ml at 37°C. IPTG was added to 4 x 10-4 M and the culture was incubated for additonal 1.5 hours at 37°C. The cells were harvested and the cellular crude extract was prepared as described in Example I. The cellular extract was applied to a cross-linked amylose column and affinity chromatography was performed as described in Example I. More than 99% of (OD 280) absorbing material in the cellular crude extract passed through cross-linked amylose column. Less than 1% of OD 280 absorbing material bound to the column could be eluted with the maltose buffer. Pst I restriction enzymatic activity was found in the pass through fraction and in the maltose buffer eluted fractions. High levels of non-specific DNAase were found in the pass through fraction but not in the maltose buffer eluted fractions. The pass through fractions consisting of the main protein peak were pooled and applied onto another cross-linked amylose column. Neither protein nor DNAase acitivity, including Pst I restriction like activity, were found to be retained by the column. In contrast, when the Pst I restriction like enzymatic activity in the maltose eluted fractions was pooled, dialysed and reapplied onto another cross-linked amylose column, all of the activity was retained by column and could be eluted with maltose buffer.

### Polyacrylamide Gel Electrophoresis

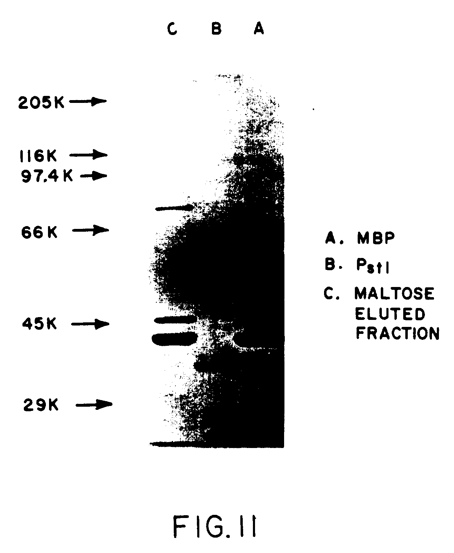

The fractions consisting of the main protein peak and the maltose eluted peak were pooled seperately. The maltose eluted pool was concentrated 25-50 fold as described in Example I. The pooled samples above were used for SDS polyacrylamide gel electrophoresis as described in Example I. The results are shown in Figure 11. Three proteins were eluted with the maltose buffer as determined by the SDS gel. The topmost band represents a protein whose molecular weight is estimated at 78 K daltons corresponding to that deduced from the sequence of the MalE-PstI gene-fusion. The lowest band comigrated with native maltose binding protein and was believed to represent the product of the Mal E gene of the host cell. It is also possible that this represents the degraded product from the hybrid polypeptide, formed as a protease resistant domain in the hybrid polypeptide. The third band which migrated slightly slower than either MBP or Pst I proteins may be degradation products.

### Example III

### Preparation of Immobilized Protein Bioreactor.

Ten milliliters of late log phase culture of strain SF1362 harboring plasmid pCG325 was harvested by centrifugation. The cell pellet was suspended in 2 ml. of buffer (10mM TriS-HCI pH 7.2). Crude extract was prepared as described in Example I. The cell extract was applied to a 0.6 x 2.5 cm cross-linked amylose column, and washed with buffer as in Example I.

### Cleavage of ONPG by the Bioreactor.

The bioreactor column was equilibrated with Z buffer as in Example I at room temperature. 500 ml of Z buffer containing 0.1% ONPG was applied to the column at room temperature with a flow rate of 0.5 ml/min. The pass through fraction was collected and the conversion to ONPG to ONP and free sugar was determined to be greater than 95%. After use the bioreactor may washed with Z buffer and stored at 4 degrees centigrade. The bioreactor can be reused multiple times.

## Claims

1. A method for producing and purifying a target protein molecule comprising:
(a) constructing a DNA expression vector which expresses a hybrid polypeptide in a transformed host cell, the hybrid polypeptide comprising the target protein molecule and a non-enzymatic biologically functional sugar binding protein, having a specific affinity for a substrate which binds to the non-enzymatic biologically functional sugar binding protein; and
(b) introducing the expression vector into an appropriate host cell and expressing the hybrid polypeptide;
(c) contacting the hybrid polypeptide produced by the transformed cell with the substrate to which the non-enzymatic biologically functional sugar binding protein binds; and
(d) recovering the target protein molecule.

2. The method of claim 1, wherein the DNA encoding the hybrid polypeptide contains a linking DNA fragment which links the DNA encoding the protein molecule with the DNA encoding the non-enzymatic biologically functional sugar binding protein.

3. The method of claim 1, wherein the non-enzymatic biologically functional sugar binding protein is maltose binding protein and the substrate is selected from the group consisting of maltose, maltodextrins and macromolecular alpha (1→4) linked glucans.

4. The method of c!aim 3, wherein the substrate is crosslinked amylose.

5. The method of claim 1, comprising the further step of releasing the hybrid polypeptide from the substrate by contacting the bound hybrid polypeptide with a substance which displaces the hybrid polypeptide.

6. The method of claim 1, wherein the substrate is contained within an affinity column.

7. The method of claim 1, comprising the further step of cleaving the protein molecule from the hybrid polypeptide.

8. The method of claim 1 or 2 wherein :
the hybrid polypeptide comprises the protein molecule, a maltose binding protein or portion thereof having a specific affinity for a substrate which binds to the maltose binding protein, and a linking sequence interposed between said protein molecule and said maltose binding protein or portion thereof, said linking sequence having a Factor Xa protease cleavage site.

9. A fusion vector which comprises:
(a) a DNA fragment coding for a non-enzymatic biologically functional sugar binding protein, the non-enzymatic biologically functional sugar binding protein having a specific affinity for a substrate which binds to the non-enzymatic biologically functional sugar binding protein; and
(b) a DNA fragment which codes for a linking sequence for linking the DNA coding for the non-enzymatic biologically functional sugar binding protein or portion thereof with a target protein molecule.

10. The fusion vector of claim 9, wherein the non-enzymatic biologically functional sugar binding protein is maltose binding protein and the substrate is selected from the group consisting of maltose, maltodextrins and macromolecular alpha (1→4) linked glucans.

11. The fusion vector of claim 9, wherein the linking sequence comprises one or more restriction sites.

12. The fusion vector of claim 9, wherein the linking sequence codes for a polypeptide which is recognized and cleaved by a proteolytic agent.

13. The fusion vector of claim 9, wherein the linking sequence codes for a spacer polypeptide which separates the non-enzymatic biologically functional sugar binding protein from the target protein molecule.

14. The fusion vector of claim 9, comprising the plasmid pCG150 obtainable from the American Type Culture Collection Deposit No. 67345.

15. A fusion vector according to claim 9 for constructing an expression vector which expresses a maltose binding protein fused to a protein molecule to be purified, comprising:
(a) a DNA fragment coding for the maltose binding protein or biologically active portion thereof, the maltose binding protein having a specific affinity for a substrate which binds to the maltose binding protein; and
(b) a DNA fragment which codes for a linking sequence having a Factor Xa protease cleavage site, wherein said DNA fragment is adapted for linking the DNA coding the maltose binding protein with the DNA coding for the protein molecule.

16. A DNA expression vector for producing a purified target protein molecule, which upon expression produces a non-enzymatic biologically functional sugar binding protein fused to the target protein molecule comprising:
(a) a DNA fragment coding for the non-enzymatic biologically functional sugar binding protein, the sugar binding protein having a specific affinity for a substrate which binds to the sugar binding protein; and
(b) a DNA fragment coding for the target protein molecule.

17. The expression vector of claim 16, wherein the non-enzymatic biologically functional sugar binding protein is maltose binding protein and the substrate is selected from the group consisting of maltose, maltodextrins and macromolecular (1→4) linked glucans.

18. The expression vector of claim 16, wherein a DNA fragment coding for a linking sequence is interposed between the DNA encoding the non-enzymatic biologically functional sugar binding protein and the DNA encoding the protein molecule.

19. The expression vector of claim 18, wherein the linking sequence comprises one or more restriction sites.

20. The expression vector of claim 18, wherein the linking sequence codes for a polypeptide which is recognized and cleaved by a proteolytic agent.

21. The expression vector of claim 18, wherein the linking sequence codes for a spacer polypeptide which separates the binding protein from the protein molecule expressed by the expression vector.

22. The DNA expression vector of claim 16 or 18, which upon expression produces a maltose binding protein fused to the protein molecule, comprising:
a DNA fragment coding for the maltose binding protein or biologically active portion thereof, the maltose binding protein having a specific affinity for a substrate which binds to the maltose binding protein; and
a linking DNA fragment coding for a linking sequence interposed between said first and second DNA fragments, wherein said linking sequence contains a Factor Xa protease cleavage site.

## Patentansprüche

1. Verfahren zum Herstellen und Reinigen eines Zielproteinmoleküls, umfassend:
(a) Konstruieren eines DNA-Expressionsvektors, welcher ein Hybridpolypeptid in einer transformierten Wirtszelle exprimiert, wobei das Hybridpolypeptid das Zielproteinmolekül und ein nicht-enzymatisches biologisch funktionelles zuckerbindendes Protein umfaßt, das eine spezifische Affinität für ein Substrat aufweist, welches an das nicht-enzymatische biologisch funktionelle zuckerbindende Protein bindet; und
(b) Einführen des Expressionsvektors in eine geeignete Wirtszelle und Exprimieren des Hybridpolypeptids;
(c) In-Kontakt-Bringen des Hybridpolypeptids, welches von der transformierten Zelle produziert wurde, mit dem Substrat, an welches das nicht-enzymatische biologisch funktionelle zuckerbindende Protein bindet; und
(d) Gewinnen des Zielproteinmoleküls.

2. Verfahren nach Anspruch 1, wobei die DNA, welche das Hybridpolypeptid codiert, ein Verknüpfungs-DNA-Fragment enthält, welches die DNA, die das Proteinmolekül codiert, mit der DNA, die das nicht-enzymatische biologisch funktionelle zuckerbindende Protein codiert, verknüpft.

3. Verfahren nach Anspruch 1, wobei das nicht-enzymatische biologisch funktionelle zuckerbindende Protein Maltosebindendes Protein ist und das Substrat ausgewählt wird aus der Gruppe bestehend aus: Maltose, Maltodextrinen und makromolekularen alpha(1→4)-verknüpften Glucanen.

4. Verfahren nach Anspruch 3, wobei das Substrat quervernetzte Amylose ist.

5. Verfahren nach Anspruch 1, welches den weiteren Schritt des Freisetzens des Hybridpolypeptids von dem Substrat durch In-Kontakt-Bringen des gebundenen Hybridpolypeptids mit einer Substanz, welche das Hybridpolypeptid verdrängt, umfaßt.

6. Verfahren nach Anspruch 1, wobei das Substrat innerhalb einer Affinitätssäule enthalten ist.

7. Verfahren nach Anspruch 1, welches den weiteren Schritt des Abspaltens des Proteinmoleküls von dem Hybridpolypeptid umfaßt.

8. Verfahren nach Anspruch 1 oder 2, wobei das Hybridpolypeptid das Proteinmolekül, ein Maltose-bindendes Protein oder einen Teil davon mit einer spezifischen Affinität für ein Substrat, welches an das Maltose-bindende Protein bindet, und eine Verknüpfungssequenz umfaßt, die zwischen dem Proteinmolekül und dem Maltose-bindenden Protein oder einem Teil davon, eingefügt ist, wobei die Verknüpfungssequenz eine Faktor Xa-Protease-Spaltungsstelle aufweist.

9. Fusionsvektor, welcher umfaßt:
(a) ein DNA-Fragment, welches für ein nicht-enzymatisches biologisch funktionelles zuckerbindendes Protein codiert, wobei das nicht-enzymatische biologisch funktionelle zuckerbindende Protein eine spezifische Affinität zu einem Substrat aufweist, welches an das nicht-enzymatische biologisch funktionelle zuckerbindende Protein bindet; und
(b) ein DNA-Fragment, welches für eine Verknüpfungssequenz codiert, um die DNA, welche für das nicht-enzymatische biologisch funktionelle zuckerbindende Protein oder einen Teil davon codiert, mit einem Zielproteinmolekül zu verknüpfen.

10. Fusionsvektor nach Anspruch 9, wobei das nicht-enzymatische biologisch funktionelle zuckerbindende Protein Maltose-bindendes Protein ist und das Substrat ausgewählt wird aus der Gruppe bestehend aus: Maltose, Maltodextrinen und makromolekularen alpha(1-4)-verknüpften Glucanen.

11. Fusionsvektor nach Anspruch 9, wobei die Verknüpfungssequenz eine oder mehrere Restriktionsstellen umfaßt.

12. Fusionsvektor nach Anspruch 9, wobei die Verknüpfungssequenz für ein Polypeptid codiert, welches durch ein proteolytisches Mittel erkannt und gespalten wird.

13. Fusionsvektor nach Anspruch 9, wobei die Verknüpfungssequenz für ein Spacer-Polypeptid codiert, welches das nicht-enzymatische biologisch funktionelle zuckerbindende Protein von dem Zielproteinmolekül trennt.

14. Fusionsvektor nach Anspruch 9, welcher das Plasmid pCG150 umfaßt, welches von der American Type Culture Collection unter der Hinterlegungsnr. 67345 erhältlich ist.

15. Fusionsvektor nach Anspruch 9 zum Konstruieren eines Expressionsvektors, welcher ein Maltose-bindendes Protein exprimiert, das mit einem zu reinigenden Proteinmolekül verbunden ist, umfassend:
(a) ein DNA-Fragment, welches für das Maltose-bindende Protein oder einen biologisch aktiven Teil davon codiert, wobei das Maltose-bindende Protein eine spezifische Affinität für ein Substrat aufweist, das an das Maltose-bindende Protein bindet; und
(b) ein DNA-Fragment, welches für eine Verknüpfungssequenz mit einer Faktor Xa-Protease-Spaltungsstelle codiert, wobei das DNA-Fragment angepaßt ist, um die DNA, die das Maltose-bindende Protein codiert, mit der DNA, die für das Proteinmolekül codiert, zu verknüpfen.

16. DNA-Expressionsvektor zum Herstellen eines gereinigten Zielproteinmoleküls, welches unter Expression ein nicht-enzymatisches biologisch funktionelles zuckerbindendes Protein produziert, das mit dem Zielproteinmolekül verbunden ist, umfassend:
(a) ein DNA-Fragment, welches für das nicht-enzymatische biologisch funktionelle zuckerbindende Protein codiert, wobei das zuckerbindende Protein eine spezifische Affinität zu einem Substrat aufweist, das an das zuckerbindende Protein bindet; und
(b) ein DNA-Fragment, welches für das Zielproteinmolekül codiert.

17. Expressionsvektor nach Anspruch 16, wobei das nicht-enzymatische biologisch funktionelle zuckerbindende Protein Maltose-bindendes Protein ist und das Substrat ausgewählt wird aus der Gruppe bestehend aus: Maltose, Maltodextrinen und makromolekularen alpha(1→4)-verknüpften Glucanen.

18. Expressionsvektor nach Anspruch 16, wobei ein DNA-Fragment, das für eine Verknüpfungssequenz codiert, zwischen der DNA, die das nicht-enzymatische biologisch funktionelle zuckerbindende Protein codiert, und der DNA, die das Proteinmolekül codiert, eingefügt ist.

19. Expressionsvektor nach Anspruch 18, wobei die Verknüpfungssequenz eine oder mehrere Restriktionsstellen umfaßt.

20. Expressionsvektor nach Anspruch 18, wobei die Verknüpfungssequenz für ein Polypeptid codiert, das durch ein proteolytisches Mittel erkannt und gespalten wird.

21. Expressionsvektor nach Anspruch 18, wobei die Verknüpfungssequenz für ein Spacer-Polypeptid codiert, welches das bindende Protein von dem Proteinmolekül, das durch den Expressionsvektor exprimiert wird, trennt.

22. DNA-Expressionsvektor nach Anspruch 16 oder 18, welcher unter Expression ein Maltose-bindendes Protein produziert, das mit dem Proteinmolekül verbunden ist, umfassend:
ein DNA-Fragment, welches für das Maltose-bindende Protein oder einen biologisch aktiven Teil davon codiert, wobei das Maltose-bindende Protein eine spezifische Affinität für ein Substrat aufweist, das an das Maltose-bindende Protein bindet; und
ein Verknüpfungs-DNA-Fragment, welches für eine Verknüpfungssequenz codiert, die zwischen dem ersten und zweiten DNA-Fragment eingefügt ist, wobei die Verknüpfungssequenz eine Faktor Xa-Protease-Spaltungsstelle enthält.

## Revendications

1. Méthode pour la production et la purification d'une molécule de protéine cible comprenant les étapes suivantes :
(a) la construction d'un vecteur d'expression d'ADN qui exprime un polypeptide hybride dans une cellule hôte transformée, le polypeptide hybride comprenant la molécule de protéine cible, et une protéine non enzymatique fixatrice de sucre biologiquement fonctionnelle, ayant une affinité spécifique pour un substrat qui se fixe à une protéine non enzymatique biologiquement fonctionnelle fixatrice de sucre ; et
(b) l'introduction du vecteur d'expression dans un hôte cellulaire approprié et l'expression du polypeptide hybride ;
(c) la mise en contact du polypeptide hybride produit par la cellule transformée avec le substrat auquel la protéine non enzymatique biologiquement fonctionnelle fixatrice de sucre se fixe ; et
(d) la récupération de la molécule de protéine cible.

2. Méthode selon la revendication 1, où l'ADN codant pour le polypeptide hybride contient un fragment d'ADN de liaison qui relie l'ADN codant pour la molécule de protéine avec l'ADN codant pour la protéine non enzymatique biologiquement fonctionnelle fixatrice de sucre.

3. Méthode selon la revendication 1, où la protéine non enzymatique biologiquement fonctionnelle fixatrice de sucre est la protéine fixant le maltose et le substrat est choisi parmi le groupe constitué du maltose, des maltodextrines et de glycanes macromoléculaires liés en alpha (1- 4).

4. Méthode selon la revendication 3, où le substrat est l'amylose réticulée.

5. Méthode selon la revendication 1, comprenant l'étape supplémentaire de relarguage du polypeptide hybride du substrat par la mise en contact du polypeptide hybride fixé avec une substance qui déplace le polypeptide hybride.

6. Méthode selon la revendication 1 où le substrat est contenu dans une colonne d'affinité.

7. Méthode selon la revendication 1, comprenant l'étape supplémentaire de clivage de la molécule de protéine du polypeptide hybride.

8. Méthode selon la revendication 1 ou 2, où le polypeptide hybride comprend une molécule de protéine, une protéine fixant le maltose ou un fragment de celle-ci ayant une affinité spécifique pour un substrat qui se fixe à la protéine fixant le maltose, et une séquence de liaison interposée entre ladite molécule de protéine et ladite protéine fixant le maltose ou son fragment, ladite séquence de liaison ayant un site de clivage de la protéase Facteur Xa.

9. Vecteur de fusion comprenant :
(a) un fragment d'ADN codant pour une protéine non enzymatique biologiquement fonctionnelle fixatrice de sucre, la protéine non enzymatique biologiquement fonctionnelle fixatrice du sucre ayant une affinité spécifique pour un substrat qui se fixe à la protéine non enzymatique biologiquement fonctionnelle fixatrice de sucre ; et
(b) un fragment d'ADN qui code pour une séquence de liaison pour relier l'ADN codant pour la protéine non enzymatique biologiquement fonctionnelle fixatrice de sucre ou une portion de celle-ci avec une molécule de protéine cible.

10. Vecteur de fusion selon la revendication 9, où la protéine non enzymatique biologiquement fonctionnelle fixatrice de sucre est la protéine fixant le maltose et le substrat est choisi parmi le groupe constitué du maltose, des maltodextrines et des glycanes macromoléculaires liés en alpha (1-4).

11. Vecteur de fusion selon la revendication 9, où la séquence de liaison comprend un ou plusieurs sites de restriction.

12. Vecteur de fusion selon la revendication 9, où la séquence de liaison code pour un polypeptide qui est reconnu et clivé par un agent protéolytique.

13. Vecteur de fusion selon la revendication 9, où la séquence de liaison code pour un polypeptide espaceur qui sépare la protéine non enzymatique biologiquement fonctionnelle fixatrice de sucre de la molécule de protéine cible.

14. Vecteur de fusion selon la revendication 9, comprenant le plamisde pCG150 déposé à l'American Type Culture Collection sous le numéro 67345.

15. Vecteur de fusion selon la revendication 9, pour la construction d'un vecteur d'expression qui exprime une protéine fixatrice de maltose fusionnée à une molécule de protéine devant être purifiée, comprenant :
(a) un fragment d'ADN codant pour la protéine fixatrice de maltose ou une portion de celle-ci biologiquement active, la protéine fixatrice de maltose ayant une affinité spécifique pour un substrat qui se fixe à la protéine fixatrice du maltose ; et
(b) un fragment d'ADN qui code pour une séquence de liaison ayant un site de clivage pour la protéase Facteur Xa où ledit fragment d'ADN est adapté pour relier l'ADN codant pour la protéine fixatrice de maltose avec l'ADN codant pour la molécule de protéine.

16. Vecteur d'expression d'ADN pour la production d'une molécule de protéine cible purifiée, qui produit lors de son expression une protéine non enzymatique biologiquement fonctionnelle fixatrice de sucre fusionnée à une molécule de protéine cible comprenant :
(a) un fragment d'ADN codant pour une protéine non enzymatique biologiquement fonctionnelle fixatrice de sucre, la protéine fixatrice de sucre ayant une affinité spécifique pour un substrat qui se fixe à la protéine fixatrice de sucre, et
(b) un fragment d'ADN codant pour une molécule de protéine cible.

17. Vecteur d'expression selon la revendication 16 où la protéine non enzymatique biologiquement fonctionnelle fixatrice de sucre est la protéine fixant le maltose et le substrat est choisi parmi le groupe constitué du maltose, des maltodextrines et de glycanes macromoléculaires liés en alpha (1-4).

18. Vecteur d'expression selon la revendication 16 où un fragment d'ADN codant pour une séquence de liaison est interposé entre l'ADN codant pour la protéine non enzymatique biologiquement fonctionnelle fixatrice de sucre et l'ADN codant pour la molécule de protéine.

19. Vecteur d'expression selon la revendication 18 où la séquence de liaison comprend un ou plusieurs sites de restriction.

20. Vecteur d'expression selon la revendication 18 où la séquence de liaison code pour un polypeptide qui est reconnu et clivé par un agent protéolytique.

21. Vecteur d'expression selon la revendication 18 où la séquence de liaison code pour un polypeptide espaceur qui sépare la protéine fixatrice de la molécule de protéine exprimée par le vecteur d'expression.

22. Vecteur d'expression d'ADN selon la revendication 16 ou 18 qui, lors de son expression, produit une protéine fixatrice de maltose fusionnée à la molécule comprenant :
un fragment d'ADN codant pour la protéine fixatrice de maltose ou une portion de celle-ci biologiquement fonctionnelle, la protéine fixant le maltose ayant une affinité spécifique pour un substrat qui se fixe à la protéine fixant le maltose ; et
un fragment d'ADN de liaison codant pour une séquence de liaison interposée entre lesdits premier et second fragments d'ADN ou ladite séquence de liaison contient un site de clivage pour la protéase facteur XA.
